# EUROPÄISCHE PATENTSCHRIFT

(11) **EP 1 018 500 B1**
(45) Veröffentlichungstag und Bekanntmachung des Hinweises auf die Patenterteilung: **11.06.2003**
(21) Anmeldenummer: 99123941.9
(22) Anmeldetag: 03.12.1999
(51) Int. Cl.: C07C 29/76, B01D 1/22, C07C 29/80

(54) **Verfahren zur Isolierung von Glykolen**
Process for the isolation of glycols
Procédé pour l'isolation de glycols

(30) Priorität: 03.12.1998 DE 19855911
(43) Veröffentlichungstag der Anmeldung: 12.07.2000
(73) Patentinhaber: BASF AKTIENGESELLSCHAFT, 67056 Ludwigshafen (DE)
(72) Erfinder: Vansant, Frans, Dr., 02920 Kalmthout (BE); De Hert, Jozef, 02040 Antwerpen 4 (BE); Köffer, Dieter, Dr., 69488 Birkenau (DE); Theis, Gerhard, Dr., 67133 Maxdorf (DE); Terjung, Winfried, 46539 Dinslaken (DE)
(74) Vertreter: Isenbruck, Günter, Dr.

(56) Entgegenhaltungen:
- CH-A- 547 765
- DE-A- 19 602 640
- US-A- 4 822 926

## Beschreibung

Die Erfindung betrifft ein Verfahren zur Herstellung und Isolierung von Glykolen sowie einen Dünnschichtverdampfer, der bei der Durchführung des Verfahrens eingesetzt wird.

Ethylenoxid (im folgenden mit EO bezeichnet) und Ethylenglykol sind wichtige Zwischenprodukte der industriellen Chemie. Wichtige Folgeprodukte des Ethylenoxids (EO) sind insbesondere Ethylenglykol, Glykolether, Ethanolamine, Ethylencarbonat und Polyethoxylate. Ethylenglykol wird vorwiegend zur Herstellung von Polyestern, Polyurethanen, Dioxan, Weichmachern und als Frostschutzmittel eingesetzt.

EO wird heute großtechnisch meist durch Direktoxidation von Ethylen an Silberoxidkatalysatoren mit molekularem Sauerstoff oder mit Luft hergestellt. Als Nebenprodukt dieser stark exothermen Reaktion entsteht zu einem Großteil durch Totaloxidation Kohlendioxid.

Industriell durchgeführte Herstellungsverfahren von EO sind beispielsweise in Ullmanns Encyclopedia of Industrial Chemistry, Fifth Edition, Carl Hanser Verlag (München), Vol. A 10, pages 117ff beschrieben. Danach enthält ein heute üblicher EO-Reaktor den Katalysator, fest angeordnet, in einem Rohrbündel mit mehreren 1000 Röhren, durch die das Reaktionsgemisch in einem Kreisgasstrom geführt wird. Als Wärmeüberträger zirkuliert zwischen den Röhren eine siedende Flüssigkeit, beispielsweise Wasser. Ethylen und Sauerstoff werden in dem durch den Reaktor geleiteten Kreisgasstrom vorgelegt, der neben den Reaktanden Inertgase und das Nebenprodukt der Ethylentotaloxidation, Kohlendioxid, enthält. Der gekühlte Reaktoraustrag wird gegebenenfalls zunächst mit einer NaOH-Lösung gequencht, um organische Säuren, insbesondere Ameisensäure und Essigsäure, sowie schwersiedende Nebenkomponenten auszuwaschen. Diese Komponenten können über einen "Quenchbleed" aus des Kreisgassystem ausgeschleust werden. In einem EO-Absorber, der in der Regel bei ca. 16 bar und ca. 25 bis 40 °C betrieben wird, wird das EO mit einem großen Waschwasserstrom (zur Absorption von 1 t EO aus 25 t Kreisgas sind ca. 35 t Waschwasser notwendig) nahezu vollständig aus dem Kreisgas ausgewaschen. Der Druck im Absorber wird dabei durch den Druck im Kreisgassystem vorgegeben. Im Kreisgas verbleiben typischerweise 5 bis 100 ppm EO. Das Kreisgas wird, nach einer Pottaschewäsche zur Entfernung von Kohlendioxid, erneut mit Ethylen und Sauerstoff angereichert und dem EO-Reaktor zugeführt. Die im Absorber anfallende wässrige Lösung enthält neben ca. 3 Gew.-% EO auch Spuren der anderen im Kreisgas enthaltenen Komponenten. Das EO wird anschließend in einem EO-Desorber (EO-Stripper) aus dem beladenen Waschwasser desorbiert. Die anfallenden Stripperbrüden enthalten ca. 50 Gew.-% Wasser und ca. 50 Gew.-% EO sowie die im beladenen Waschwasser enthaltenen Gase. Das abgereicherte Waschwasser wird nach der Kühlung als Kreiswasser zurück zum EO-Desorber geführt. Da im EO-Desorber das EO teilweise zu Glykolen hydrolysiert wird, werden diese über den sogenannten Glykolbleed aus dem EO-Desorber ausgeschleust. Die Desorberbrüden werden zunächst kondensiert Anschließend werden aus dem Kondensat in einer sogenannten Lightendkolonne die gelösten Gase ausgestrippt and zum Kreisgassystem zurückgeführt. Die so gewonnene gasfreie, ca. 50 %-ige wässrige EO-Lösung wird teilweise in einer Destillationskolonne zu Rein-EO aufdestilliert bzw. nach Wasserzugabe in einem Glykolreaktor zu einer wässrigen Glykollösung umgesetzt.

Der Hydrolysereaktor wird üblicherweise bei Temperaturen von 120 bis 250 °C und Drücken von 30 bis 40 bar betrieben. Das Hydrolyseprodukt wird zunächst entwässert (auf einen Restwassergehalt von 100 bis 200 ppm) und anschließend in die verschiedenen Glykole in reiner Form aufgetrennt. Bei der Hydrolyse entsteht zunächst vorwiegend Monoethlyenglykol, das anschließend teilweise mit EO zu Di-Tri-, und Polyethylenglykolen weiterreagieren kann. Da Monoethylenglykol (nachstehend vereinfachend als Ethyenglykol bezeichnet) prinzipiell im gesamten Prozeß das einzige vorkommende Glykol sein kann, kann das Wort Glykole (im Plural) im folgenden auch ausschließlich Monoethylenglykol (Ethylenglykol) bedeuten.

Die Entwässerung erfolgt in der Regel in einer Kaskade von druckgestaffelten Kolonnen, mit abnehmendem Druck. Aus Gründen der Wärmeintegration wird in der Regel nur der Sumpfverdampfer der ersten Druckkolonne mit Fremddampf beheizt, alle weiteren Druckkolonnen dagegen mit den Brüden der jeweils voranstehenden Kolonne. Je nach Wassergehalt des Hydrolysereaktoraustrags und des Druck-/Temperaturniveaus des im Sumpfverdampfer der ersten Kolonne eingesetzten Fremddampfs besteht die Druckentwässerungskaskade aus 2 bis 7 Kolonnen. An die Druckentwässerung schließt sich eine Vakuumentwässerung an. Die entwässerte Glykolmischung wird in mehreren Kolonnen in die Reinstoffe zerlegt. Die Produkte Monoethylenglykol, Di-, Tri- und gegebenenfalls Tetraethylenglykol werden jeweils als Kopfprodukt abgezogen, alle weiteren höheren Glykole fallen in Form eines Gemischs unter der Bezeichnung Polyethylenglykole als Sumpfprodukt der letzten Kolonne an. Das Hauptprodukt dieser Aufarbeitung ist in der Regel Monoethylenglykol (Ethylenglykol).

Der EO-haltige Quenchbleed kann wegen der Toxizität des EO nicht ohne weiteres entsorgt werden. Darüber hinaus besteht ein wirtschaftliches Interesse, die enthaltenen Wertstoffe EO und Glykol wiederzugewinnen. Das EO kann z.B. in einem Quenchbleed-Stripper desorbiert und dem EO-Desorber zugeführt werden. Alternativ kann das EO im Quenchbleed in einem Hydrolysereaktor bei Temperaturen von 160 bis 230°C und Drücken von 20 bis 35 bar in Glykole umgewandelt werden. Die so erhältliche wässrige Glykollösung kann problemlos entsorgt werden.

Alternativ können gemäß der US-A-4,822,926 die Glykole nach Aufkonzentrierung in einem Flasher und Salzabtrennung in einer Zentrifuge wiedergewonnen werden. Nachteil dieser Methode ist der Anfall eines Feststoffes, der im allgemeinen schwieriger als Flüssigkeiten zu handhaben ist.

Da in dem EO-Absorber-Desorber-Wasserkreislauf das EO teilweise zu Glykolen hydrolysiert wird, wird ein Teilstrom des Sumpfablaufes des EO-Desorbers ausgeschleust und aufkonzentriert, wobei die entstehenden Brüden dem EO-Desorber zurückgeführt werden können (beschrieben in der DD-A-235 635). Die aufkonzentrierte Glykollösung, der sogenannte Glykolbleed, wird einem Flasher zugeführt, wobei die Glykole zum Teil zurückgewonnen werden. Der salz- und glykolhaltige Sumpfstrom wird entsorgt.

Die DE-A 196 02 640 beschreibt ein Verfahren zur Verdampfung einer verdampfbare oxidationsempfindliche Verbindungen enthaltenden Flüssigkeit in einem Verdampfer, in dem die Flüssigkeit zur Verdampfung in Kontakt mit einer erhitzten festen Oberfläche gebracht wird, derart, daß ein direkter Kontakt zwischen einer gebildeten Dampfphase und der erhitzten festen Oberfläche weitgehend vermieden wird. Der Verdampfer kann ein Fallfilmverdampfer, Umlaufverdampfer, Röhrenverdampfer, Durchlaufverdampfer, Dünnschichtverdampfer, Kletterfilmverdampfer, Plattenverdampfer, vorzugsweise ein Fallfilmverdampfer sein. Die verdampfbare oxidationsempfindliche Flüssigkeit ist vorzugsweise ein Glykol, insbesondere Ethylenglykol, wobei die Flüssigkeit Wasser enthält.

Die CH-A 547 765 offenbart ein Verfahren zur Reinigung von verunreinigtem Glykol, bei dem das Glykol zweimal hintereinander einer Verdampfung ausgesetzt und der dabei entstehende Glykoldampf einer Rektifikation unterworfen wird. Das Verfahren wird in zwei hintereinandergeschalteten Verdampfern durchgeführt, in denen eine Rektifikationskolonne nachgeschaltet sowie ein Wärmeaustauscher vorgeschaltet ist. Wenigstens der zweite Verdampfer ist ein Dünnschichtverdampfer mit mechanischen Mitteln zur Erzeugung der dünnen Schicht.

Der vorliegenden Erfindung lag die Aufgabe zugrunde, ein Verfahren bereitzustellen, bei dem die während des Prozesses entstehenden Glykole als Reinstoffe gewonnen werden. Dabei sollen die entsprechenden, neben Glykolen in der Regel noch Salze und Wasser enthaltenden Gemische so aufgetrennt werden, daß Produkte hoher Qualität erhalten werden. Außerdem soll gewährleistet werden, daß möglichst alle Glykole zurückgewonnen werden, so daß die bei dem Prozeß anfallenden, zu entsorgenden Rückstände reduziert werden. Das Verfahren soll effektiv und kostengünstig sein.

Die Lösung dieser Aufgabe geht aus von dem Verfahren zur Isolierung von Glykolen aus einem Glykole, Wasser und Salze enthaltenden flüssigen Gemisch durch
(a) Verdampfung zumindest einer Teilmenge des Wassers und der Glykole, die im Gemisch enthalten sind, unter. Abscheidung eines von Salz befreiten, Glykole und Wasser aufweisenden, flüssigen oder gasförmigen Mediums,
(b) Entwässerung des Mediums und
(c) Isolierung der Glykole aus dem entwässerten Medium.

Das erfindungsgemäße Verfahren ist dann dadurch gekennzeichnet, daß zur Durchführung der Stufe (a) ein Dünnschichtverdampfer eingesetzt wird, der ein Sumpfablaufsystem aufweist, das periodisch gespült wird.

In einer bevorzugten Ausführungsform fällt das Glykole, Wasser und Salze enthaltende flüssige Gemisch bei der Aufarbeitung einer aus der katalytischen Umsetzung von Ethylen mit Sauerstoff resultierenden, EO aufweisenden Reaktionsmischung an, wobei während der Aufarbeitung, vor der Entstehung des flüssigen Gemischs, zumindest eine Teilmenge des EO zu Ethylenglykol hydrolysiert wird.

Erfindungsgemäß wird auch ein Verfahren zur Herstellung von Glykolen bereitgestellt. Bei diesem wird durch katalytische Umsetzung von Ethylen mit Sauerstoff eine EO aufweisende Reaktionsmischung erzeugt, die anschließend aufgearbeitet wird. Dabei fällt ein Glykole, Wasser und Salze enthaltendes flüssiges Gemisch an. Das Herstellungsverfahren ist dadurch gekennzeichnet, daß dieses Gemisch gemäß dem letzteren Verfahren (Verfahren zur Isolierung von Glykolen) aufgetrennt wird.

Außerdem ist erfindungsgemäß ein Dünnschichtverdampfer zur Durchführung der Stufe (a) des Verfahrens zur Isolierung von Glykolen vorgesehen, der folgende Einrichtungen enthält:
I. ein Ableitungsrohr für das am Kopf anfallende, von Salzen befreite, Glykole und Wasser enthaltende, flüssige oder gasförmige Medium
II. einen Sumpfablauf,
III. einen Zulauf,
IV. ein Einleitungsrohr für Spülmittel,
V. zwei Einleitungsrohre für Inertgas,
VI. einen Spülablauf,
VII. einen Ablaufbehälter,
VIII. einen Sammelbehälter,
IX. ein Ablaufventil,
X. ein Spülwasserventil,
XI. ein Behälterablaufventil,
XII. ein Spülablaufventil und
XIII. ein Entlüftungsventil.

Der zur Durchführung der Stufe (a) des erfindungsgemäßen Isolationsverfahrens eingesetzte Dünnschichtverdampfer wird in der Regel bei Temperaturen von 160 bis 220 °C, bevorzugt bei 180 bis 200 °C betrieben, wobei der Druck, der in dem Dünnschichtverdampfer vorliegt, meist zwischen 30 und 200 mbar, bevorzugt zwischen 40 und 80 mbar beträgt.

Der Dünnschichtverdampfer weist ein Sumpfablaufsystem auf, das periodisch gespült wird. In der Regel wird das Sumpfablaufsystem mit Wasser gespült.

Die Glykole aus den wässrigen, salzhaltigen Lösungen können effektiv, kostengünstig und umweltfreundlich abgetrennt werden. Es handelt sich insbesondere um Glykole, die während der Aufarbeitung in einem Quench-Bleed-Konzentrator (Glykolanteil in diesem bis mindestens 50 Gew.-%, bevorzugt bis mindestens 80 Gew.-%) und in einem Glykolbleed-Flasher anfallen. Das Glykole, Wasser und Salze enthaltende flüssige Gemisch, das dem erfindungsgemäßen Isolationsvefahren zugrundeliegt, entsteht bevorzugt durch Vereinigung der Sumpfausträge des Quench-Bleed-Konzentrators und des Glykolbleed-Flashers. In der Regel ist das Glykole, Wasser und Salze enthaltende flüssige Gemisch frei oder nahezu frei (weniger als 0,1 Gew.% EO ist enthalten) von EO. Der Anteil der Glykole in dem Glykole, Wasser und Salze enthaltenden flüssigen Gemisch beträgt meist mindestens 50 Gew.%, bevorzugt mindestens 80 Gew.%.

Die Brüden des Dünnschichtverdampfers werden kondensiert und man erhält (gemäß Stufe (a) des erfindungsgemäßen Isolationsverfahrens) das von Salz befreite, Glykole und Wasser aufweisende, flüssige oder gasförmige Medium, das der Vakuumentwässerung des Hauptglykolstroms zugeführt wird. Der salzhaltige flüssige Rückstand aus dem Dünnschichtverdampfer wird entsorgt. Dabei wird das Sumpfablaufsystem des Dünnschichtverdampfers gespült (in der Regel periodisch mit Wasser), so daß eine Salzabsetzung vermieden wird. Die Betriebsbedingungen garantieren eine optimale Glykolwiedergewinnung und eine problemlose Wirkung dieses Verfahrensschrittes. Die benötigte Wassermenge, um den Rückstand problemlos aus dem Dünnschichtverdampfer zu entfernen, kann in der Regel auf 10 bis 20 % der Rückstandsmenge begrenzt werden, so daß die so entstandene Rückstandsmischung durch Verbrennung entsorgt werden kann. Der Rückstand kann aber auch in einer biologischen Kläranlage entsorgt werden. In diesem Fall ist es nicht nötig, die Wassermenge zu minimieren.

Im folgenden soll eine bevorzugte Ausführungsform der Erfindung näher beschrieben werden:

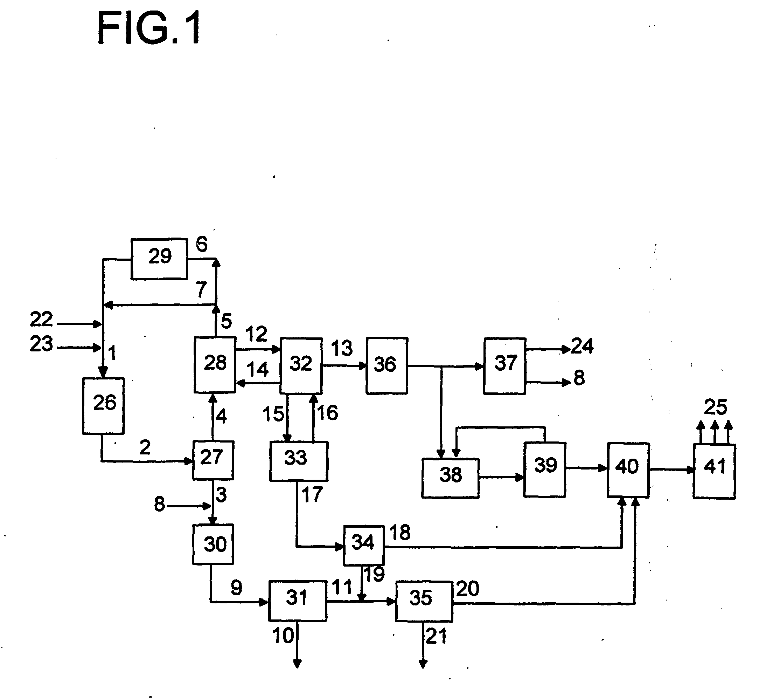

Fig. 1 der Zeichnung stellt eine schematische Darstellung eines EO/Glykol-Verfahrens dar. Maschinen und Apparate wie zum Beispiel Pumpen, Wärmetauscher und Behälter, die für die Erläuterung der Erfindung nicht wesentlich sind, sind nicht aufgeführt.

Ethylen und Sauerstoff werden in einem Kreisgas 1 vorgelegt, das neben den Reaktanden Inertgase, zum Beispiel Stickstoff, das Nebenprodukt der Ethylentotaloxidation, Kohlendioxid und Verunreinigungen der Reaktanden, zum Beispiel Ethan und Argon enthält. Die Reaktion verläuft in einem Reaktor 26 (alternativ auch in mehreren Reaktoren) an einem silberhaltigen Katalysator. Neben EO und den Nebenprodukten der Totaloxidation, Kohlendioxid und Wasser werden auch in kleinen Mengen Nebenkomponenten, wie Acetaldehyd, Formaldehyd und organische Säuren wie Ameisensäure und Essigsäure, gebildet. Der Reaktoraustrag 2 wird zunächst abgekühlt und anschließend einem Quench 27 zugeführt. In dem Quench 27 kondensiert der größere Teil des gebildeten Reaktionswassers aus. Dem Quenchumlauf wird eine NaOH-Lösung zugegeben, - um die organischen Säuren, insbesondere Ameisensäure und Essigsäure, auszuwaschen. Außerdem wird dadurch ein Teil des Kohlendioxids unter Bildung von Natriumsalzen umgesetzt. Prinzipiell kann die Salzbildung auch mit anderen Basen erfolgen. Bei den gebildeten Salzen handelt es sich insbesondere um Bicarbonate, Carbonate, Formiate und Acetate. Ebenfalls wird ein Teil des gebildeten EO ausgewaschen, und ein kleiner Teil des EO wird in Glykol umgewandelt. Der Quenchumlauf wird üblicherweise bei Temperaturen von 25 bis 50 °C und Drücken von 12 bis 20 bar betrieben. Ein Teil des Quenchumlaufes wird als Quenchbleed-Strom 3 ausgeschleust. Ein typischer Quenchbleed enthält 1 bis 3 Gew.-% EO; 1 bis 3 Gew.-% Glykol; 0,2 bis 1 Gew.-% Natriumsalze (insbesondere Natriumbicarbonat, Natriumcarbonat, Natriumformiat und Natriumacetat), außerdem kleinere Mengen von Nebenkomponenten wie Formaldehyd und Acetaldehyd, sowie gelöste Gase aus dem Kreisgas, zum Beispiel Ethylen und Methan.

Das auf diese Weise gereinigte Reaktionsgas aus dem Quench 27 wird dem EO-Absorber 28 zugeführt, wobei in diesem bei Temperaturen von 25 bis 45 °C und Drücken von 12 bis 20 bar das EO mit einem großen Wasserstrom nahezu vollständig ausgewaschen wird. Das von EO befreite Kreisgas 5 wird in zwei Ströme geteilt. Der erste Teilstrom 6 wird einer Pottaschewäsche zur Entfernung von Kohlendioxid zugeführt und anschließend mit dem zweiten Teilstrom des Kreisgases 7 vereinigt. Nach Anreicherung mit Ethylen und Sauerstoff wird das gesamte Kreisgas 1 dem EO-Reaktor wieder zugeführt.

Die gelösten Gase in dem Quenchbleed können zunächst wahlweise in einem Flasher abgetrennt und zum Beispiel dem EO-Desorber 32 oder der Lightendskolonne 36 zugeführt werden. Dies ist allerdings nicht unbedingt erforderlich (und deshalb auch nicht in der Figur dargestellt).

Der Quenchbleed wird nach Aufheizung einem Quench-Bleed-Hydrolyse-Reaktor 30 zugeführt. Die benötigten Temperaturen, Drücke und Verweilzeiten sind vergleichbar mit den Betriebsparametern in dem Glykol-Reaktor 38 zur Herstellung von Glykolen. Es muß sichergestellt werden, daß EO vollständig in Glykole umgewandelt wird und daß keine Phasentrennung auftritt. Besonders vorteilhaft ist das Verfahren in einer Anlage, in der ein Teil des EO zu Rein-EO aufdestilliert wird, wonach zusätzlich der aldehydhaltige Strom 8 aus der Rein-EO-Destillation dem Quenchbleed-Hydrolyse-Reaktor 30 zugeführt wird.

Der Quenchbleed-Reaktoraustrag 9 wird in einem Quench-Bleed-Konzentrator 31 aufkonzentiert, wobei das Wasser als Kopfstrom 10 abgezogen wird. Die Aufkonzentrierung erfolgt zweckmäßigerweise in einer Destillationskolonne oder in einer Kaskade von druckgestaffelten Kolonnen mit Wärmeintegration.

Der Quenchbleed-Konzentrator 31 wird so betrieben, daß die Glykolkonzentration im Sumpfaustrag 11 mindestens 50 Gew.-%, bevorzugt mindestens 80 Gew.-% beträgt.
Die im EO-Absorber 28 anfallende wässrige EO-Lösung 12 wird einem EO-Desorber 32 zugeführt, in dem das EO aus dem beladenen Waschwasser desorbiert wird. Die anfallenden Stripperbrüden 13 enthalten ca. 50 Gew.-% Wasser und ca. 50 Gew.-% EO sowie die im beladenen Waschwasser enthaltenden Gase. Das abgereicherte Waschwasser 14 wird nach Kühlung als Kreiswasser zurück zum EO-Absorber 28 geführt. Da in dem EO-Absorber-Desorber-Kreislauf das EO teilweise zu Glykolen hydrolysiert wird, wird ein Teilstrom 15 des Sumpfablaufes des EO-Desorbers ausgeschleust und in dem Glykolbleed-Konzentrator 33 aufkonzentriert, wobei die entstehenden Brüden 16 in den EO-Desorber 32 zurückgeführt werden. Die aufkonzentrierte Glykollösung, der sogenannte Glykolbleed 17, wird dem Glykolbleed-Flasher 34 zugeführt.

Der Glykolbleed-Konzentrator 33 wird üblicherweise bei Temperaturen von 130 bis 140 °C und Drücken von 1,5 bis 2,5 bar betrieben. Die Glykolkonzentration des Sumpfablaufes beträgt bevorzugt 40 bis 60 Gew.-%. Der Glykolbleed 17 wird dem Glykolbleed-Flasher 34 zugeführt, in dem Wasser und Glykol verdampft werden. Der Kopfstrom 18 des Glykolbleed-Flashers wird bevorzugt dampfförmig der Vorrichtung zur Glykol-Vakuumentwässerung 40 zugeführt. Der Glykolbleed-Flasher 34 wird üblicherweise bei Temperaturen von 110 bis 140 °C und Drücken von 300 bis 400 mbar betrieben. Der Sumpfaustrag des Glykolbleed-Flashers 19 wird mit dem Sumpfaustrag des Quench-Bleed-Konzentrators 11 vermischt und dem Dünnschichtverdampfer 35 zugeführt.

Dünnschichtverdampfung wird für kontinuierliche Destillation, insbesondere zur Verdampfung temperaturempfindlicher Substanzen aus hochsiedenden Rückständen und für die Aufkonzentration von temperaturlabilen Stoffen eingesetzt. Hierbei verteilt man die Flüssigkeit durch Abrieselnlassen (FallfilmVerdampfer), Einwirkung von Zentrifugalkraft (spezielle Ausführungsform eines Rotationsverdampfers), besonders konstruierte Wischer (Filmtruder) oder anderen Techniken zu dünnen Schichten (Größenordnung der Schichtdicke in der Regel ca. 0,1 mm) auf den (in der Regel) beheizten Flächen. Dünne Flüssigkeitsschichten ermöglichen eine schnelle Verdampfung, so daß die enthaltenen Komponenten nur kurzzeitig den (in der Regel) höheren Temperaturen im Verdampfer ausgesetzt sind. Die Verweilzeit, die Temperatur und der Druck (Vakuum) werden entsprechend der jeweiligen Trennaufgabe ausgelegt. Dabei sind zur Schonung der betreffenden Substanzen möglichst niedrige Temperaturen und kurze Verweilzeiten zu bevorzugen. Neben Vorrichtungen, die im allgemeinen technischen Sprachgebrauch als Dünnschichtverdampfer bezeichnet werden, werden erfindungsgemäß alle Verdampfungs-Vorrichtungen als Dünnschichtverdampfer verstanden, die nach dem vorstehend beschriebenen Prinzip arbeiten.

Ein Dünnschichtverdampfer erweist sich als besonders geeignet, um eine salzhaltige Glykollösung in ein salzfreies, dampfförmiges Glykol-Wasser-Gemisch (dieses wird durch das Ableitungsrohr 20 abgeführt) und einen stark konzentrierten salzhaltigen Sumpfablauf 21 aufzutrennen. Ein solcher Dünnschichtverdampfer arbeitet bei Temperaturen von 160 bis 220 °C, bevorzugt von 180 bis 200 °C und bei Drücken von 40 bis 200 mbar, bevorzugt von 60 bis 90 mbar. Eine schonende Trennung durch Dünnschichtverdampfung ist deshalb sinnvoll, da Glykole in Anwesenheit von Sauerstoffs und/oder enthaltener Salze leicht qualitätsmindernde Nebenprodukte bilden, insbesondere wenn die Glykole längere Zeit höheren Temperaturen ausgesetzt sind.

Der Sumpfablauf 21 des Dünnschichtverdampfers 35 enthält in der Regel 10 bis 30 Gew.-%, bevorzugt 15 bis 25 Gew.-% Salze. Besonders effektiv kann ein Gemisch aus den beiden vorstehend beschriebenen Bleedströmen (einerseits der Sumpfaustrag des Quenchbleed-Konzentrators 11 und andererseits der Sumpfaustrag des Glykolbleed-Flashers 19) in einem Dünnschichtverdampfer 35 aufgetrennt werden. Der Sumpfablauf 21 des Dünnschichtverdampfers 35 kann nach Vermischung mit 10 bis 20 Gew.-% Wasser abgepumpt und gelagert werden. Das Gemisch ist geeignet, um in einer Rückstandsverbrennung verbrannt zu werden. Es ist ebenfalls möglich, dieses Gemisch in einer biologischen Kläranlage zu entsorgen. In diesem Fall ist es nicht nötig, die Wassermenge zu minimieren. Das dampfförmige Glykol-Wasser-Gemisch wird nach Kondensation der Vorrichtung zur Glykol-Vakuumentwässerung 40 zugeführt. Anschließend wird die entwässerte Glykolmischung destillativ in die entsprechenden Produkte aufgetrennt (in Vorrichtung 41).

### Technische Ausführung des Dünnschichtverdampfers 35:

Dünnschichtverdampfer werden, wie bereits einleitend beschrieben, üblicherweise zur Destillation und Eindampfung von temperaturempfindlichen Stoffen eingesetzt. Ein typischer erfindungsgemäß einsetzbarer Dünnschichtverdampfer 35 weist ein senkrecht stehendes, von außen beheiztes Rohr auf, auf dessen Innenfläche das zu verdampfende Produkt von einem Rotor als dünne Schicht ausgebreitet wird. Das Produkt tritt oberhalb der Heizzone in den Verdampfer ein und fließt infolge der mechanischen Verteilung durch den Rotor als Flüssigkeitsfilm über die Heizfläche. Die leichtsiedenden Komponenten verdampfen, während der Rückstand nach unten abläuft. Die Brüdendämpfe strömen durch einen Tropfenabscheider, der sich im Kopf des Verdampfers befindet und werden in einem separat angeordneten Kondensator kondensiert. Zur Beheizung der Verdampferfläche ist ein Doppelmantel vorgesehen, in dem die Beheizung mit Heizdampf erfolgt. Für die mechanische Flüssigkeitsverteilung ist ein Rotor mit pendelnd aufgehängten Wischerblättern aus Metall eingebaut. Beim Betrieb werden die Wischerblätter infolge der Fliehkraft gegen die Verdampferwand gedrückt, was eine gleichmäßige Produktverteilung und eine intensive Flüssigkeitsdurchmischung des Flüssigkeitsfilmes bewirkt.

Der Dünnschichtverdampfer soll nicht nur die nahezu vollständige Verdampfung (Tri- und Tetraglykole lassen sich schwerer Verdampfen - eine nahezu vollständige Verdampfung bezieht sich daher in der Regel auf etwa 10 % der Glykole, die im Sumpf verbleiben) der Glykole aus der glykol- und salzhaltigen Lösung gewährleisten, sondern auch die Salze (insbesondere Natriumbicarbonat, Natriumcarbonat, Natriumformiat und Natriumacetat) als eine pumpfähige Mischung bereitstellen. Bei einem Dünnschichtverdampfer, der nach dem vorstehend beschriebenen Prinzip arbeitet, ist dies jedoch nicht der Fall, denn Salze können sich im Bereich des Ablaufs abscheiden und Verkrustungen ausbilden, so daß der Sumpfaustrag aufgrund von Verstopfungen nicht mehr problemlos abgeführt werden kann. Der kontinuierliche Betrieb der Dünnschichtverdampfung muß beim Auftreten solcher Verstopfungen eingestellt werden, damit entsprechende Reinigungsarbeiten eingeleitet werden können.

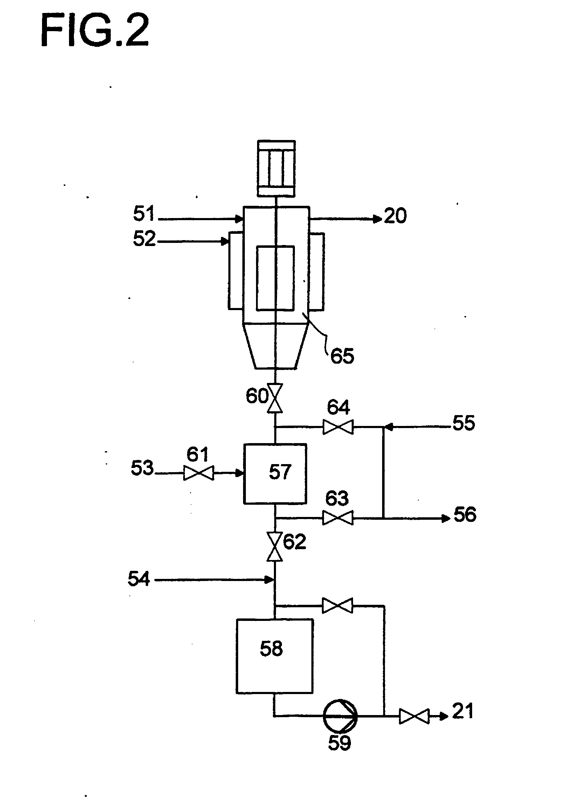

Um zu vermeiden, daß das Sumpfaustragsystem durch auskristallisierende Salze verstopft, wird die Ablaufleitung mittels einer speziellen Spülvorrichtung (diese ist in Fig. 2 der Zeichnung dargestellt) periodisch gespült. Dazu wird der Sumpfablauf zunächst in einem Ablaufbehälter 57 aufgefangen, welcher sich dabei allmählich auffüllt. Nach Erreichen eines bestimmten Füllstandes wird das Ablaufventil 60 geschlossen und das Vakuum im Ablaufbehälter 57 durch Öffnen des Behälterablaufventils 62 zum Sammelbehälter 58 aufgehoben (der Sammelbehälter 58 steht unter Normaldruck oder leichtem Überdruck). Anschließend wird das Entlüftungsventil 64 geöffnet, und der Inhalt des Ablaufbehälters 57 läuft in den Sammelbehälter 58 ab. Der Inhalt des Sammelbehälters 58 wird zweckmäßigerweise mittels einer Pumpe 59 zirkuliert, um diesen homogen zu halten und um mögliche Salzabsetzung zu verhindern. Der Inhalt wird zur Zwischenlagerung und weiteren Verwertung, zum Beispiel der Verbrennung, kontinuierlich oder diskontinuierlich abgeführt.

Nun wird der Ablaufbehälter 57 gespült. Zweckmäßigerweise verwendet man dazu Wasser, bevorzugt warmes Wasser oder Dampfkondensat. Zum Einleiten des Spülvorganges werden das Behälterablaufventil 62 geschlossen, das Spülablaufventil 63 und das Spülwasserventil 61 geöffnet. Das warme Spülwasser, das durch das Einleitungsrohr für Spülmittel 53 zugeführt wird, durchströmt nun den Ablaufbehälter 57 und entfernt auf diese Weise Salzabsetzungen. Der Spülablauf kann ohne Probleme einer biologischen Kläranlage zugeführt werden. Spüldauer und Wasserdurchfluß werden nach Bedarf so eingestellt, daß die benötigte Wassermenge so gering wie möglich ist. Je nach Belastung des Dünnschichtverdampfers 35 und Salzgehalt sind eine Wassermenge von 10 bis 50 und eine Spülzeit von 1 bis 2 Minuten ausreichend. Um Verstopfungen in der Ablaufleitung zum Sammelbehälter 58 zu vermeiden, wird diese Leitung durch Öffnen des Behälterablaufventils 62 (anstelle des Spülablaufventils 63) periodisch gespült. Vorzugsweise erfolgt dies alle 10 bis 20 Spüldurchgänge.

Nach Ablauf einer Vorspülzeit wird das Spülablaufventil 63 geschlossen, und im weiteren Verlauf der Spülphase wird das Wasser über den Ablaufbehälter 57 und die Ablaufleitung durch die Entlüftungsleitung und durch das Entlüftungsventil 64 gespült. Anschließend wird das Spülwasserventil 61 geschlossen, und um den Ablaufbehälter zu entleeren, das Spülablaufventil 63 geöffnet. Restliches Spülwasser läßt man durch öffnen des Behälterablaufventils 62 ablaufen. Danach werden alle Ventile wieder geschlossen. Durch Öffnen des Ablaufventils 60 wird der Ablaufbehälter wieder evakuiert, und der Ablaufzyklus kann erneut beginnen.

Um das Eindringen von Luft zu vermeiden, sind die Ablaufleitungen zum Sammelbehälter und zum Spülmittelablauf mit Inertgasspülungen (in der Regel Stickstoffspülung) versehen. Die Zufuhr von Inertgas erfolgt über die Einleitungsrohre (54; 55).

Vorteilhaft ist eine automatisierte Ablaufsteuerung dieser Ablauf- und Spülvorgänge.

### Beispiel:

In der nachstehenden Tabelle sind die Daten eines bevorzugten Verfahrens zur großtechnischen Gewinnung von EO, und Glykolen in einer gekoppelten, erfindungsgemäß arbeitenden Anlage zusammengefaßt. Die Werte gelten für eine Anlage mit einer Kapazität von 300 000 jato EO, wovon 100 000 jato in reines EO (Rein-EO) aufdestilliert werden und das restliche EO in ca. 275 000 jato Glykole umgewandelt wird.

Aus den in der Tabelle aufgeführten Ergebnissen wird deutlich, daß die erfindungsgemäße Aufarbeitung der Quenchbleed- und Glykolbleed-Ströme effektiv erfolgt. Die gesamten Verluste der Quenchbleed- und Glykolbleed-Ströme sind umgerechnet bezogen auf EO nur 0,12 Gew.%. Die Wertstoffe in den Bleedströmen werden dem Hauptglykolstrom zugeführt und gelangen dadurch in die Reinglykole.

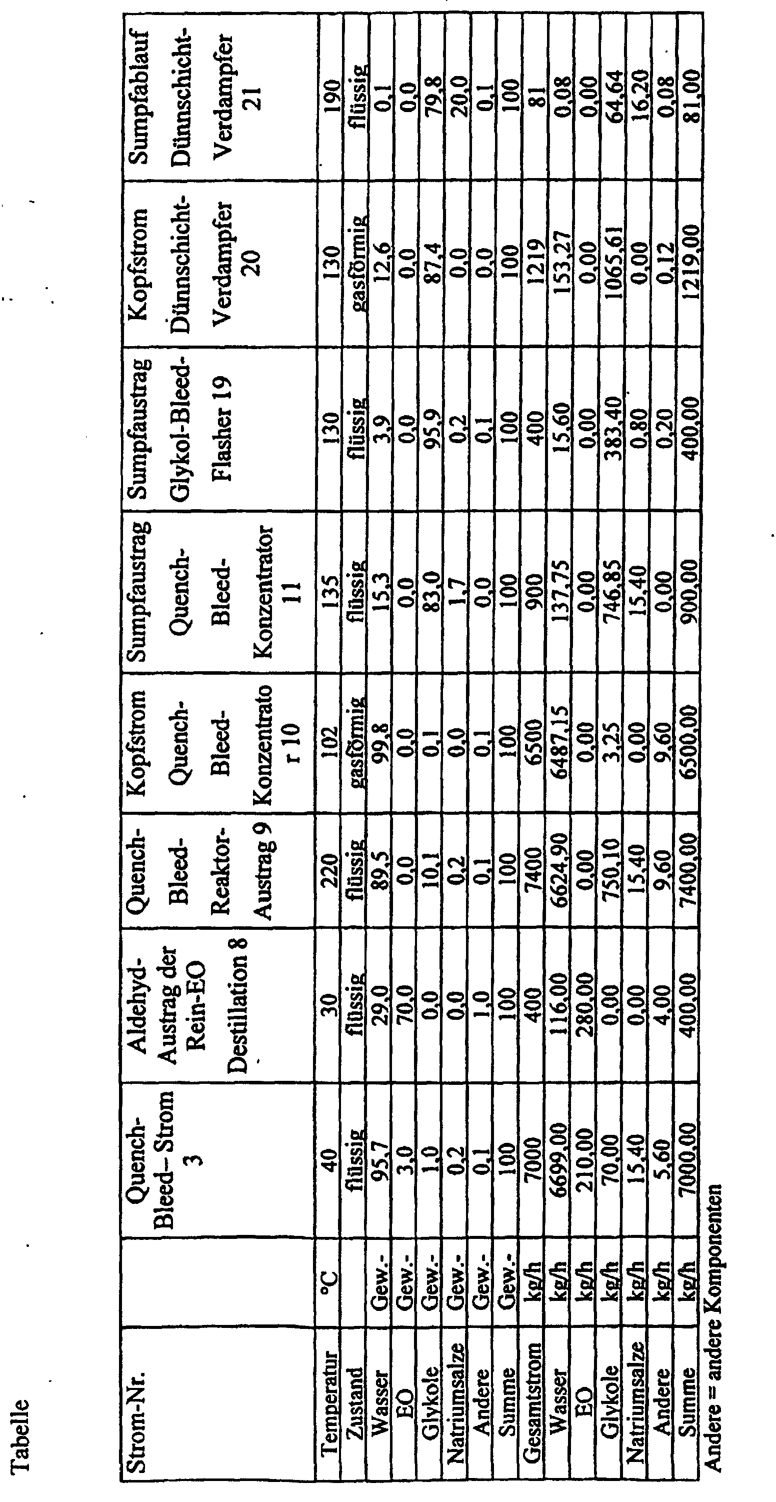

### Bezugszeichenliste:

- 1: in den Reaktor eingeführter Kreisgasstrom
- 2: aus dem Reaktor ausgetragener Strom
- 3: Quenchbleed-Strom
- 4: Strom des aus dem Quench stammenden, gereinigten Reaktionsgases
- 5: Strom des dem EO-Absorber abgeführten, von EO befreiten Kreisgases
- 6: Kreisgasstrom, der dem Pottaschewäscher zugeführt wird
- 7: zweiter Teilstrom des Kreisgases
- 8: aldehydhaltiger Strom aus der EO-Rein-Destillation
- 9: Strom des Quenchbleed-Reaktoraustrags
- 10: Kopfstrom des Quench-Bleed-Konzentrators
- 11: Sumpfaustrag des Quench-Bleed-Konzentrators
- 12: im EO-Absorber anfallende wässrige EO-Lösung
- 13: Stripperbrüden
- 14: abgereichertes Waschwasser
- 15: Teilstrom des Sumpfablaufs des EO-Desorbers
- 16: Brüdenstrom des Glykol-Bleed-Konzentrators
- 17: Glykolbleed
- 18: Kopfstrom des Glykol-Bleed-Flashers
- 19: Sumpfaustrag des Glykol-Bleed-Flashers
- 20: Ableitungsrohr für das am Kopf des Dünnschichtverdampfers anfallende, von Salzen befreite, Glykole und Wasser enthaltende, flüssige oder gasförmige Medium
- 21: Sumpfablauf des Dünnschichtverdampfers
- 22: Einleitungsrohr für Ethylen
- 23: Einleitungsrohr für Sauerstoff
- 24: Ableitungsrohr für reines EO
- 25: Ableitungsrohre für reine Glykole
- 26: Reaktor zur Herstellung von EO
- 27: Quench
- 28: EO-Absorber
- 29: Pottasche-Wäscher
- 30: Quench-Bleed-Hydrolyse-Reaktor
- 31: Quench-Bleed-Konzentrator
- 32: EO-Desorber
- 33: Glykol-Bleed-Konzentrator
- 34: Glykol-Bleed-Flasher
- 35: Dünnschichtverdampfer
- 36: Lightends-Kolonne
- 37: Kolonne zur EO-Reindestillation
- 38: Glykol-Reaktor
- 39: Vorrichtung zur Glykol-Druckentwässerung
- 40: Vorrichtung zur Glykol-Vakuumentwässerung
- 41: Vorrichtung zur Destillation der entwässerten Glykolmischung
- 51: Zulauf in den Dünnschichtverdampfer
- 52: Dampfstrom zur Beheizung des Reaktormantels
- 53: Einleitungsrohr für Spülmittel
- 54: Einleitungsrohr für Inertgas
- 55: Einleitungsrohr für Inertgas
- 56: Spülablauf
- 57: Ablaufbehälter
- 58: Sammelbehälter
- 59: Pumpe
- 60: Ablaufventil
- 61: Spülwasserventil
- 62: Behälterablaufventil
- 63: Spülablaufventil
- 64: Entlüftungsventil
- 65: Innenraum des Dünnschichtverdampfers

## Patentansprüche

1. Verfahren zur Isolierung von Glykolen aus einem Glykole, Wasser und Salze enthaltenden flüssigen Gemisch durch
(a) Verdampfung zumindest einer Teilmenge des Wassers und der Glykole, die im Gemisch enthalten sind, unter Abscheidung eines von Salz befreiten, Glykole und Wasser aufweisenden, flüssigen oder gasförmigen Mediums,
(b) Entwässerung des Mediums und
(c) Isolierung der Glykole aus dem entwässerten Medium,
**dadurch gekennzeichnet, daß** zur Durchführung der Stufe (a) ein Dünnschichtverdampfer (35) eingesetzt wird, der ein Sumpfablaufsystem: aufweist, das periodisch gespült wird.

2. Verfahren nach Anspruch 1, **dadurch gekennzeichnet, daß** das Glykole, Wasser und Salze enthaltende flüssige Gemisch bei der Aufarbeitung einer aus der katalytischen Umsetzung von Ethylen mit Sauerstoff resultierenden, EO aufweisenden Reaktionsmischung anfällt und dabei während der Aufarbeitung, vor der Entstehung des flüssigen Gemischs, zumindest eine Teilmenge des EO zu Ethylenglykol hydrolysiert wird.

3. Verfahren nach Anspruch 2, **dadurch gekennzeichnet, daß** während der Aufarbeitung Glykole in einem Quench-Bleed-Konzentrator (31) und in einem Glykolbleed-Flasher (34) anfallen und daß das Glykole, Wasser und Salze enthaltende flüssige Gemisch durch Vereinigung der Sumpfausträge (11; 19) des Quench-Bleed-Konzentrators (31) und des Glykolbleed-Flashers (34) entsteht.

4. Verfahren nach einem der Ansprüch 1 bis 3, **dadurch gekennzeichnet, daß** das Glykole, Wasser und Salze enthaltende flüssige Gemisch frei oder nahezu frei von EO ist.

5. Verfahren nach einem der Ansprüch 1 bis 4, **dadurch gekennzeichnet, daß** der Anteil der Glykole in dem Glykole, Wasser und Salze enthaltenden flüssigen Gemisch mindestens 50 Gew.%, bevorzugt mindestens 80 Gew.%, beträgt.

6. Verfahren nach einem der Ansprüche 1 bis 5, **dadurch gekennzeichnet, daß** der zur Durchführung der Stufe (a) eingesetzte Dünnschichtverdampfer (35) bei Temperaturen von 160 bis 220 °C, bevorzugt von 180 bis 200 °C, betrieben wird.

7. Verfahren nach einem der Ansprüche 1 bis 6, **dadurch gekennzeichnet, daß** der Druck, der in dem Dünnschichtverdampfers (35) vorliegt, zwischen 30 und 200 mbar, bevorzugt zwischen 40 und 80 mbar, beträgt.

8. Verfahren nach einen der Ansprüche 1 bis 7, **dadurch gekennzeichnet, daß** das Sumpfablaufsystem mit Wasser gespült wird.

9. Verfahren zur Herstellung von Glykolen durch katalytische Umsetzung von Ethylen mit Sauerstoff unter Entstehung einer EO aufweisenden Reaktionsmischung und anschließende Aufarbeitung der Reaktionsmischung, wobei ein Glykole, Wasser und Salze enthaltendes flüssiges Gemisch anfällt, **dadurch gekennzeichnet, daß** dieses Gemisch gemäß dem Verfahren nach Anspruch 1 aufgetrennt wird.

10. Dünnschichtverdampfer (35) zur Durchführung der Stufe (a) nach einem der Ansprüche 1 bis 9 enthaltend folgende Einrichtungen:
I. ein Ableitungsrohr (20) für das am Kopf anfallende, von Salzen befreite, Glykole und Wasser enthaltende, flüssige oder gasförmige Medium,
II. einen Sumpfablauf (21),
III. einen Zulauf (51),
IV. ein Einleitungsrohr (53) für Spülmittel,
V. zwei Einleitungsrohre (54; 55) für Inertgas,
VI. einen Spülablauf (56),
VII. einen Ablaufbehälter (57),
VIII. einen Sammelbehälter (58),
IX. ein Ablaufventil (60),
X. ein Spülwasserventil (61),
XI. ein Behälterablaufventil (62),
XII. ein Spülablaufventil (63) und
XIII. ein Entlüftungsventil (64).

## Claims

1. A process for isolating glycols from a liquid mixture comprising glycols, water and salts by
(a) evaporating at least a partial amount of the water and glycols which are present in the mixture, separating off a liquid or gaseous medium which is freed from salt and comprises glycols and water,
(b) dewatering the medium and
(c) isolating the glycols from the dewatered medium,
which comprises using a thin-film evaporator (35) for carrying out stage (a), said evaporator having a bottoms outflow system which is cleaned at intervals.

2. A process as claimed in claim 1, wherein the liquid mixture comprising glycols, water and salts arises in the work-up of a reaction mixture which comprises EO and results from the catalytic reaction of ethylene with oxygen and, during the work-up, before the liquid mixture is formed, at least a partial amount of the EO is hydrolyzed to form ethylene glycol.

3. A process as claimed in claim 2, wherein, during the work-up, glycols arise in a quench bleed concentrator (31) and in a glycol bleed flasher (34) and wherein the liquid mixture comprising glycols, water and salts is formed by combining the bottoms discharges (11; 19) of the quench bleed concentrator (31) and the glycol bleed flasher (34).

4. A process as claimed in one of claims 1 to 3, wherein the liquid mixture comprising glycols, water and salts is free or virtually free of EO.

5. A process as claimed in one of claims 1 to 4, wherein the content of glycols in the liquid mixture comprising glycols, water and salts is at least 50% by weight, preferably at least 80% by weight.

6. A process as claimed in one of claims 1 to 5, wherein the thin-film evaporator (35) used to carry out stage (a) is operated at from 160 to 220°C, preferably from 180 to 200°C.

7. A process as claimed in one of claims 1 to 6, wherein the pressure present in the thin-film evaporator (35) is from 30 to 200 mbar, preferably from 40 to 80 mbar.

8. A process as claimed in one of claims 1 to 7, wherein the bottoms outflow system is cleaned with water.

9. A process for preparing glycols by catalytic reaction of ethylene with oxygen with the formation of a reaction mixture comprising EO and subsequent work-up of the reaction mixture, in which a liquid mixture comprising glycols, water and salts is produced, which comprises separating this mixture according to the process as claimed in claim 1.

10. A thin-film evaporator (35) for carrying out stage
(a) as claimed in one of claims 1 to 9, comprising the following apparatuses:
I. an outlet tube (20) for the liquid or gaseous medium which arises at the top, is freed from salts and comprises glycols and water,
II. a bottoms outflow (21),
III. a feed (51),
IV. a cleaning agent introduction tube (53),
V. two inert gas introduction tubes (54; 55),
VI. a cleaning outflow (56),
VII. an outflow vessel (57),
VIII. a collecting vessel (58),
IX. an outflow valve (60),
X. a cleaning-water valve (61),
XI. a vessel outflow valve (62),
XII. a cleaning outflow valve (63) and
XIII. a vent valve (64).

## Revendications

1. Procédé pour la séparation de glycols à partir d'un mélange liquide contenant des glycols, de l'eau et des sels, comprenant les étapes consistant à
(a) évaporer au moins une partie de l'eau et des glycols qui sont contenus dans le mélange, en séparant un milieu, liquide ou gazeux, contenant les glycols et l'eau et qui est libéré des sels,
(b) déshydrater le milieu, et
(c) isoler les glycols à partir du milieu déshydraté,
**caractérisé en ce que** l'on met en oeuvre, pour réaliser l'étape (a), un évaporateur à couche mince (35) présentant un système d'évacuation de fond qui est rincé périodiquement.

2. Procédé selon la revendication 1, **caractérisé en ce que** le mélange liquide contenant les glycols, l'eau et les sels, est produit lors du traitement d'un mélange réactionnel qui provient d'une réaction catalytique de l'éthylène avec de l'oxygène et qui contient de l'oxyde d'éthylène, et **caractérisé en ce que** l'on hydrolyse au moins une partie de l'oxyde d'éthylène en éthylèneglycol, pendant le traitement et avant que le mélange liquide ne se forme.

3. Procédé selon la revendication 2, **caractérisé en ce que** les glycols sont produits, pendant le traitement, dans un concentrateur à trempe de soutirage (31) et dans un distillateur éclair à soutirage de glycol (34), et **caractérisé en ce que** le mélange liquide contenant les glycols, l'eau et les sels est formé au moyen de l'association des produits de fond (11; 19) provenant du concentrateur à trempe de soutirage (31) et du distillateur éclair à soutirage de glycol (34).

4. Procédé selon l'une quelconque des revendications 1 à 3, **caractérisé en ce que** le mélange liquide contenant les glycols, l'eau et les sels est exempt ou bien essentiellement exempt d'oxyde d'éthylène.

5. Procédé selon l'une quelconque des revendications 1 à 4, **caractérisé en ce que** le mélange liquide contenant les glycols, l'eau et les sels présente une teneur en glycols d'au moins 50% en poids, de préférence d'au moins 80% en poids.

6. Procédé selon l'une quelconque des revendications 1 à 5, **caractérisé en ce que** l'on fait fonctionner l'évaporateur à couche mince (35), mis en oeuvre pour réaliser l'étape (a), à une température allant de 160°C à 220°C, de préférence de 180°C à 200°C.

7. Procédé selon l'une quelconque des revendications 1 à 6, **caractérisé en ce que** la pression, dans l'évaporateur à couche mince (35), va de 30 mbars à 200 mbars, de préférence de 40 mbars à 80 mbars.

8. Procédé selon l'une quelconque des revendications 1 à 7, **caractérisé en ce que** l'on rince le système d'évacuation de fond avec de l'eau.

9. Procédé pour la préparation de glycols au moyen d'une réaction catalytique de l'éthylène avec de l'oxygène, en formant un mélange réactionnel contenant de l'oxyde d'éthylène puis en traitant le mélange réactionnel, dans lequel est produit un mélange liquide contenant des glycols, de l'eau et des sels, **caractérisé en ce que** l'on sépare ce mélange selon le procédé selon la revendication 1.

10. Evaporateur à couche mince (35) pour réaliser l'étape (a), selon l'une quelconque des revendications 1 à 9, comprenant les dispositifs suivants :
I. une canalisation d'évacuation (20) pour le milieu, gazeux ou liquide, contenant les glycols et l'eau et libéré des sels, produit au niveau de la tête,
II. une évacuation de fond (21),
III. une arrivée (51),
IV. une canalisation d'alimentation (53) pour l'agent de rinçage,
V. deux canalisations d'alimentation (54; 55) pour le gaz inerte,
VI. une évacuation de rinçage (56),
VII. un récipient d'évacuation (57),
VIII. un récipient collecteur (58),
IX. une vanne d'évacuation (60),
X. une vanne pour l'eau de rinçage (61),
XI. une vanne d'évacuation du récipient (62),
XII. une vanne d'évacuation du rinçage (63), et
XIII. une vanne d'aération (64).
